# EUROPEAN PATENT SPECIFICATION

(11) **EP 4 312 873 B1**
(45) Date of publication and mention of the grant of the patent: **08.10.2025**
(21) Application number: 22715457.2
(22) Date of filing: 18.03.2022
(51) Int. Cl.: A61C 13/00, A61C 9/00, A61C 19/04

(54) **CONFIGURING DENTAL WORKFLOWS THROUGH INTELLIGENT RECOMMENDATIONS**
KONFIGURATION VON ZAHNARBEITSABLÄUFEN DURCH INTELLIGENTE EMPFEHLUNGEN
CONFIGURATION DE FLUX DE TRAVAIL DENTAIRE À TRAVERS DES RECOMMANDATIONS INTELLIGENTES

(30) Priority: 25.03.2021 US 202117211955
(43) Date of publication of application: 07.02.2024
(73) Proprietor: DENTSPLY SIRONA Inc., York, PA 17401 (US)
(72) Inventor: DERZAPF, Evgenij, 64625 Bensheim (DE); REINING, Marius, 64625 Bensheim (DE); SEIBERT, Frank, 64625 Bensheim (DE); SCHNEIDER, Hans-Christian, 64625 Bensheim (DE)
(74) Representative: Aldridge, Henry Alexander
(86) International application number: PCT/US2022/020872
(87) International publication number: WO 2022/203950

(56) References cited:
- WO-A1-2021/025296
- CA-A1- 3 098 404
- CA-C- 3 098 404
- US-A1- 2019 282 344
- US-A1- 2021 251 729

## Description

### TECHNICAL FIELD

The present invention relates to a computer implemented method, computer system, and non-transitory computer-readable storage medium for intelligent proposal of restorations and dental/workflow parameters for a restoration and machining procedure.

### BACKGROUND

Presently, technology exists to design restorations and help manufacture them. For example, dental software can traditionally be used for restoration design and manufacturing using input from dental professionals. The process includes an administration phase in which a restoration type such as single tooth restoration, bridge restoration and implant is selected for the patient. A tooth number for the restoration, restoration material and material color are then selected. After the administration, a three-dimensional (3D) scan of the patient's dental cavity is obtained with a dental scanner in an acquisition phase. In a next phase, a buccal registration of the patient's bite is obtained, margin lines are drawn, and model and insertion axes are determined. In a model axes determination, the orientation in the occlusal, mesial and buccal view are established. Further, possible undercuts in regions within a preparation margin are identified and corrected before producing a design of the restoration. In a subsequent phase, a digital design of the restoration is obtained based on tooth models in a database or biogeneric tooth models. After obtaining the design, the digital restoration is manufactured based on selected manufacturing processes including milling or grinding options.
Reference is made to US 2019/282344 A1, WO 2021/025296A1, and CA 3 098 404 A1.

### SUMMARY

The invention is as defined in the appended claims. The illustrative embodiments provide a method, system and non-transitory computer-readable storage medium for configuring a dental workflow. In an aspect herein, a computer- implement method is disclosed. The computer-implemented method comprises the steps of: receiving a three-dimensional (3D) scan of a dental cavity of a patient for a dental workflow; identifying, by a computer-aided design/computer-aided manufacturing (CAD/CAM) resource configured to automatically obtain information about the dental workflow, input data for a dental configuration recommendation module; extracting, responsive to the identifying, one or more features from the input data, the one or more features representative of a request for completing a restoration design and/or machining process, and proposing, using the dental configuration recommendation module, at least one user-specific configuration for the restoration design and/or machining process.

In another aspect herein, any combination of the following are disclosed: (i) the proposing includes at least one of (a) proposing a user-specific restoration design as a first configuration, (b) proposing at least one restoration parameter as a second configuration, and (c) proposing at least one user-specific manufacturing parameter as a third configuration. (ii) providing, using an adaptation module, a modification of dental workflow setting or of a proposed user-specific configuration from a plurality of proposed user-specific configurations and computing an acceptance decision of the modification based on a set of dependencies, (iii) recomputing, the at least one user-specific configuration upon determining that the modification is acceptable, (iv) the input data is automatically obtained from devices contained in or connected to the dental manufacturing system, (v) at least a portion of the input data is automatically retrieved from the 3D scan of the dental cavity, (vi) the dental configuration recommendation module operates as a machine learning engine, (vii) the input data is data selected from the group consisting of CAD/CAM input data, preferences and dependencies, (viii) the dental configuration recommendation module proposes restoration parameters as an output, (ix) training the dental configuration recommendation module based on information comprising static CAD/CAM input data, dynamic CAD/CAM input data and user preferences, (x) wherein the information includes historical data selected from a database of a dental manufacturing system such as a chairside restoration system, and (xi) locally or remotely providing the dental configuration recommendation module with new instructions and requirements as the dependencies.

In another aspect, not part of the claimed invention, an embodiment includes a computer usable program product. The computer usable program product includes a computer-readable storage device, and program instructions stored on the storage device.

In a further aspect, not part of the claimed invention, an embodiment includes a computer system. The computer system includes a processor, a computer-readable memory, and a computer-readable storage device, and program instructions stored on the storage device for execution by the processor via the memory.

### BRIEF DESCRIPTION OF THE DRAWINGS

Certain novel features believed characteristic of the invention are set forth in the appended claims. The invention itself, however, as well as a preferred mode of use, further objectives and advantages thereof, will best be understood by reference to the following detailed description of the illustrative embodiments when read in conjunction with the accompanying drawings, wherein:
FIG. 1 depicts a block diagram of a network of data processing systems in which illustrative embodiments may be implemented;
FIG. 2 depicts a block diagram of a data processing system in which illustrative embodiments may be implemented;
FIG. 3 depicts a block diagram of an example configuration for intelligent proposal of restorations and dental/workflow parameters for machining in accordance with an illustrative embodiment;
FIG. 4A depicts a block diagram of a restoration recommendation configuration in which illustrative embodiments may be implemented;
FIG. 4B depicts a block diagram of a manufacturing parameter recommendation configuration in which illustrative embodiments may be implemented;
FIG. 5 depicts a block diagram of a machining interface of a presentation module in accordance with an illustrative embodiment;
FIG. 6 depicts a block diagram of an example training architecture for machine-learning based recommendation engine in accordance with an illustrative embodiment;
FIG. 7 depicts a flowchart of an example process in accordance with an illustrative embodiment.

### DETAILED DESCRIPTION

The illustrative embodiments recognize that the user of every dental CAD/CAM software for restoration design and manufacturing must deal with configurations about defining a restoration type and type of machining and material for machining at the start of every new case. As far as configuring workflows for a dental case, presently, dental workflow attributes are manually selected in a software by a dental practitioner in a time-consuming process that may not be convenient for a new user. The illustrative embodiments recognize that the configuration process requires expert know-how which are constantly becoming complex and changing frequently and choosing, manufacturing or designing with a wrong parameter will result, at best, in the creation restorations that differ vastly from an expected or desired outcome, and at worst, in unsafe dental products and working conditions.

As an example, a dental practitioner, upon scanning a patient's jaws for a dental procedure, manually configures restoration and manufacturing parameters by modification of default parameters presented by a software. This is time consuming, error-prone and often not optimal for the particular patient because the practitioner may lack all of the knowledge about the parameters and their dependencies.

The illustrative embodiments thus recognize that presently available tools or solutions do not address the need to provide intelligent management of dental workflow configurations in order to provide optimized restorations in a timely manner. The illustrative embodiments used to describe the invention generally address and solve the above-described problems and other related problems by employing a machine learning engine to configure dental workflows in a patient and/or user specific manner through intelligent recommendations.

Such a configuration includes restoration parameters such as minimum wall thickness, edge thickness, edge design (chamfer or rounded, chamfer length, edge radius), spacer and the like. The configuration also includes manufacturing parameters such as type of machining device (for example, a particular milling unit or a 3D printer), a type of machining (for example, grinding, milling), a type of material (for example, feldspar, lithium disilicate, polymethacrylate, titanium, zirconium, etc.), a type or size of machining tool (for example, step bur, cylinder bur, cylinder bur EF (extra fine)) , a mode of machining (for example, optimization of triangle quality, machining time/cost (e.g. fast, normal, fine). The restoration parameters and manufacturing parameters are collectively referred to herein as dental parameters. Of note, the configuration process may also include the designing of a restoration geometry in preparation for manufacturing.

These examples of settings, configurations, parameters, properties and the like are not intended to be limiting. From this disclosure, those of ordinary skill in the art will be able to conceive many other aspects applicable towards a similar purpose, and the same are contemplated within the scope of the illustrative embodiments.

The illustrative embodiments are described with respect to certain types of data, functions, algorithms, equations, model configurations, locations of embodiments, additional data, devices, data processing systems, environments, components, and applications only as examples. Any specific manifestations of these and other similar artifacts are not intended to be limiting to the invention. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the invention, either locally at a data processing system or over a data network, within the scope of the invention.

The illustrative embodiments are described using specific code, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other dental system, structures, applications, or architectures therefor, may be used in conjunction with such embodiment of the invention within the scope of the invention. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

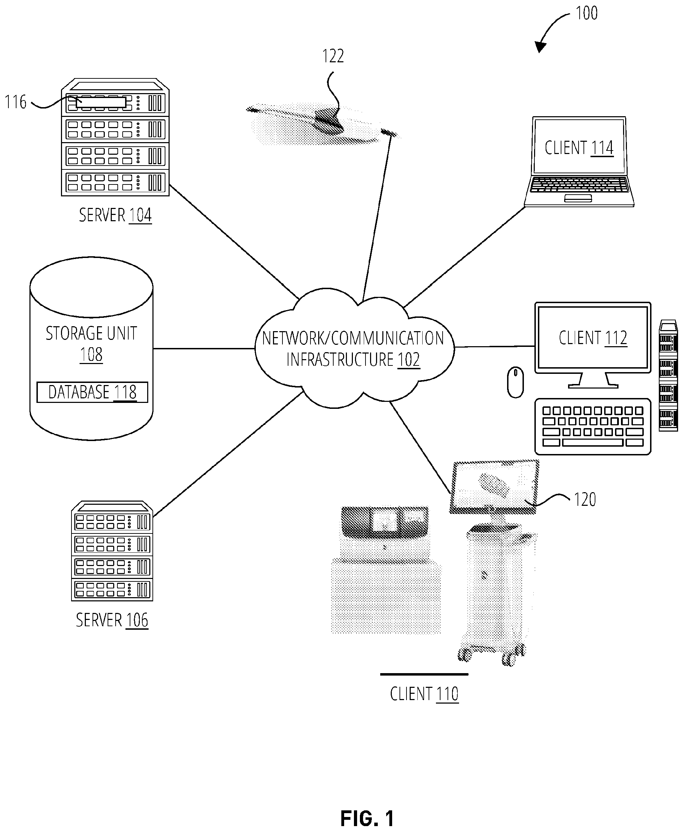

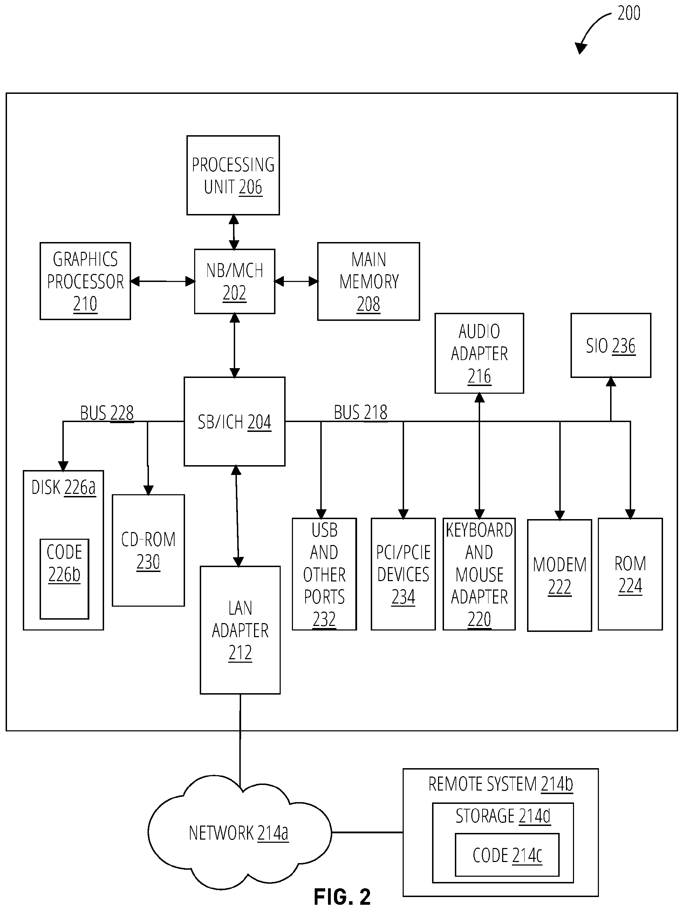

With reference to the figures and in particular with reference to FIG. 1 and FIG. 2, these figures are example diagrams of data processing environments in which illustrative embodiments may be implemented. FIG. 1 and FIG. 2 are only examples and are not intended to assert or imply any limitation with regard to the environments in which different embodiments may be implemented. A particular implementation may make many modifications to the depicted environments based on the following description.

FIG. 1 depicts a block diagram of a network of data processing systems in which illustrative embodiments may be implemented. Data processing environment is a network of computers in which the illustrative embodiments may be implemented. Data processing environment **100** includes network/communication infrastructure **102.** Network/communication infrastructure **102** is the medium used to provide communications links between various devices, databases and computers connected together within data processing environment **100.** Network/communication infrastructure **102** may include connections, such as wire, wireless communication links, or fiber optic cables.

Clients or servers are only example roles of certain data processing systems connected to network/communication infrastructure **102** and are not intended to exclude other configurations or roles for these data processing systems. Server **104** and server **106** couple to network/communication infrastructure **102** along with storage unit **108.** Software applications may execute on any computer in data processing environment **100.** Client **110,** client **112,** client **114** are also coupled to network/communication infrastructure **102.** Client **110** may be a dental manufacturing system such as a dental acquisition unit with a display and a milling unit operatively connected thereto. A data processing system, such as server **104** or server **106,** or clients (client **110,** client **112,** client **114**) may contain data and may have software applications or software tools executing thereon.

Only as an example, and without implying any limitation to such architecture, FIG. 1 depicts certain components that are usable in an example implementation of an embodiment. For example, servers and clients are only examples and do not to imply a limitation to a client-server architecture. As another example, an embodiment can be distributed across several data processing systems and a data network as shown, whereas another embodiment can be implemented on a single data processing system within the scope of the illustrative embodiments. Data processing systems (server **104,** server **106,** client **110,** client **112,** client **114**) also represent example nodes in a cluster, partitions, and other configurations suitable for implementing an embodiment.

Dental scanner **122** includes one or more sensors that measure tooth geometry and/or color by obtaining a plurality of images through projections and combining the projections to obtain a three-dimensional (3D) image. In an example, the dental scanner **122** captures data points as often as several thousand times each second, automatically registering the sizes and shapes of each tooth. It continuously sends this data to the connected computer's software, which builds it into a 3D impression of the patient's oral cavity.

A most widely used digital format is the STL (Standard Tessellation Language) format. This format describes a succession of triangulated surfaces where each triangle is defined by three points and a normal surface. STL files may describe only the surface geometry of a three-dimensional object without any representation of color, texture or other CAD model attributes. However, other file formats have been developed to record color, transparency, or texture of dental tissues (such as Polygon File Format, PLY files). Irrespective of the type of imaging technology employed, scanners or cameras project light that is then recorded as individual images and compiled by the software after recognition of POI (points of interest). For example, two coordinates (*x* and *y*) of each point are evaluated on the image, and the third coordinate (*z*) is then calculated depending on a distance from the scanner.

Client application **120** or any other application **116** implements an embodiment described herein. Client application **120** can use data from dental scanner **122** to generate restoration proposals. Client application **120** can also automatically derive input data from a CAD/CAM resource module **324** for use in proposing restoration geometries and dental parameters.

Client application **120** can also execute in any of data processing systems (server **104** or server **106,** client **110,** client **112,** client **114**), such as client application **116** in server **104** and need not execute in the same system as client **110.**

Server **104,** server **106,** storage unit **108,** client **110,** client **112,** client **114,** may couple to network/communication infrastructure **102** using wired connections, wireless communication protocols, or other suitable data connectivity. Client **110,** client **112** and client **114** may be, for example, personal computers or network computers.

In the depicted example, server **104** may provide data, such as boot files, operating system images, and applications to client **110,** client **112,** and client **114.** Client **110,** client **112** and client **114** may be clients to server **104** in this example. Client **110,** client **112** and client **114** or some combination thereof, may include their own data, boot files, operating system images, and applications. Data processing environment **100** may include additional servers, clients, and other devices that are not shown. Server **104** includes an application **116** that may be configured to implement one or more of the functions described herein for displaying a live control view in accordance with one or more embodiments.

Server **106** may include a search engine configured to search stored files such as images, 3D models of patients and preferences for a dental practice in response to a request from an operator as described herein with respect to various embodiments.

In the depicted example, data processing environment **100** may be the Internet. Network/communication infrastructure **102** may represent a collection of networks and gateways that use the Transmission Control Protocol/Internet Protocol (TCP/IP) and other protocols to communicate with one another. At the heart of the Internet is a backbone of data communication links between major nodes or host computers, including thousands of dental practices, commercial, governmental, educational, and other computer systems that route data and messages. Of course, data processing environment **100** also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment **100** may also employ a service-oriented architecture where interoperable software components distributed across a network may be packaged together as coherent business applications. Data processing environment **100** may also take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

With reference to FIG. 2, this figure depicts a block diagram of a data processing system in which illustrative embodiments may be implemented. Data processing system **200** is an example of a computer, such client **110,** client **112,** client **114** or s server **104,** server **106,** in FIG. 1, or another type of device in which computer usable program code or instructions implementing the processes may be located for the illustrative embodiments.

Data processing system **200** is described as a computer only as an example, without being limited thereto. Implementations in the form of other devices, in FIG. 1, may modify data processing system **200,** such as by adding a touch interface, and even eliminate certain depicted components from data processing system **200** without departing from the general description of the operations and functions of data processing system **200** described herein.

In the depicted example, data processing system **200** employs a hub architecture including North Bridge and memory controller hub (NB/MCH) **202** and South Bridge and input/output (I/O) controller hub (SB/ICH) **204.** Processing unit **206,** main memory **208,** and graphics processor **210** are coupled to North Bridge and memory controller hub (NB/MCH) **202.** Processing unit **206** may contain one or more processors and may be implemented using one or more heterogeneous processor systems. Processing unit **206** may be a multi-core processor. Graphics processor **210** may be coupled to North Bridge and memory controller hub (NB/MCH) **202** through an accelerated graphics port (AGP) in certain implementations.

In the depicted example, local area network (LAN) adapter **212** is coupled to South Bridge and input/output (I/O) controller hub (SB/ICH) **204.** Audio adapter **216,** keyboard and mouse adapter **220,** modem **222,** read only memory (ROM) **224,** universal serial bus (USB) and other ports **232,** and PCI/PCIe devices **234** are coupled to South Bridge and input/output (I/O) controller hub (SB/ICH) **204** through bus **218.** Hard disk drive (HDD) or solid-state drive (SSD) **226**a and CD-ROM **230** are coupled to South Bridge and input/output (I/O) controller hub (SB/ICH) **204** through bus **228.** PCI/PCIe devices **234** may include, for example, Ethernet adapters, add-in cards, and PC cards for notebook computers. PCI uses a card bus controller, while PCIe does not. Read only memory (ROM) **224** may be, for example, a flash binary input/output system (BIOS). Hard disk drive (HDD) or solid-state drive (SSD) **226**a and CD-ROM **230** may use, for example, an integrated drive electronics (IDE), serial advanced technology attachment (SATA) interface, or variants such as external-SATA (eSATA) and micro- SATA (mSATA). A super I/O (SIO) device **236** may be coupled to South Bridge and input/output (I/O) controller hub (SB/ICH) **204** through bus **218.**

Memories, such as main memory **208,** read only memory (ROM) **224,** or flash memory (not shown), are some examples of computer usable storage devices. Hard disk drive (HDD) or solid-state drive (SSD) **226**a, CD-ROM **230,** and other similarly usable devices are some examples of computer usable storage devices including a computer usable storage medium.

An operating system runs on processing unit **206.** The operating system coordinates and provides control of various components within data processing system **200** in FIG. 2. The operating system may be a commercially available operating system for any type of computing platform, including but not limited to server systems, personal computers, and mobile devices. An object oriented or other type of programming system may operate in conjunction with the operating system and provide calls to the operating system from programs or applications executing on data processing system **200.**

Instructions for the operating system, the object-oriented programming system, and applications or programs, such as application **116** and client application **120** in FIG. 1, are located on storage devices, such as in the form of codes **226**b on Hard disk drive (HDD) or solid-state drive (SSD) **226**a, and may be loaded into at least one of one or more memories, such as main memory **208,** for execution by processing unit **206.** The processes of the illustrative embodiments may be performed by processing unit **206** using computer implemented instructions, which may be located in a memory, such as, for example, main memory **208,** read only memory (ROM) **224,** or in one or more peripheral devices.

Furthermore, in one case, code **226**b may be downloaded over network **214**a from remote system **214**b, where similar code **214**c is stored on a storage device **214**d in another case, code **226**b may be downloaded over network **214**a to remote system **214**b, where downloaded code **214**c is stored on a storage device **214**d.

The hardware in FIG. 1 and FIG. 2 may vary depending on the implementation. Other internal hardware or peripheral devices, such as flash memory, equivalent non-volatile memory, or optical disk drives and the like, may be used in addition to or in place of the hardware depicted in FIG. 1 and FIG. 2. In addition, the processes of the illustrative embodiments may be applied to a multiprocessor data processing system.

In some illustrative examples, data processing system **200** may be a personal digital assistant (PDA), which is generally configured with flash memory to provide non-volatile memory for storing operating system files and/or user-generated data. A bus system may comprise one or more buses, such as a system bus, an I/O bus, and a PCI bus. Of course, the bus system may be implemented using any type of communications fabric or architecture that provides for a transfer of data between different components or devices attached to the fabric or architecture.

A communications unit may include one or more devices used to transmit and receive data, such as a modem or a network adapter. A memory may be, for example, main memory **208** or a cache, such as the cache found in North Bridge and memory controller hub (NB/MCH) **202.** A processing unit may include one or more processors or CPUs.

The depicted examples in FIG. 1 and FIG. 2 and above-described examples are not meant to imply architectural limitations. For example, data processing system **200** also may be a tablet computer, laptop computer, or telephone device in addition to taking the form of a mobile or wearable device.

Where a computer or data processing system is described as a virtual machine, a virtual device, or a virtual component, the virtual machine, virtual device, or the virtual component operates in the manner of data processing system **200** using virtualized manifestation of some or all components depicted in data processing system **200.** For example, in a virtual machine, virtual device, or virtual component, processing unit **206** is manifested as a virtualized instance of all or some number of hardware processing units **206** available in a host data processing system, main memory **208** is manifested as a virtualized instance of all or some portion of main memory **208** that may be available in the host data processing system, and Hard disk drive (HDD) or solid-state drive (SSD) **226**a is manifested as a virtualized instance of all or some portion of Hard disk drive (HDD) or solid-state drive (SSD) **226**a that may be available in the host data processing system. The host data processing system in such cases is represented by data processing system **200.**

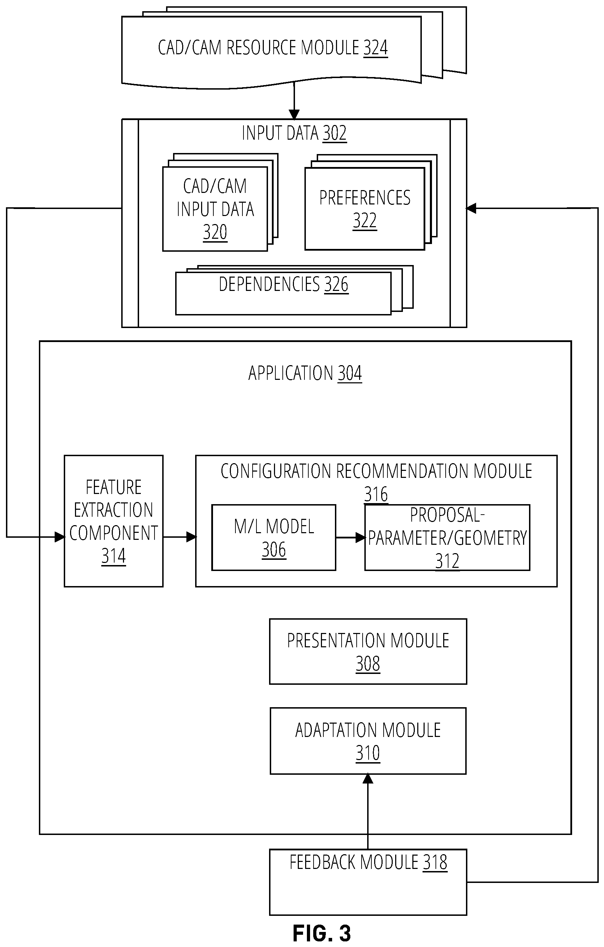

With reference to FIG. 3, this figure depicts a block diagram of an example configuration for intelligent proposal of restorations and dental/workflow parameters in accordance with an illustrative embodiment. Application **304** is an example of any of server applications **116** or client application **120** in FIG. 1, depending on the particular implementation.

Application **304** may automatically derive input data from a CAD/CAM resource module **324** (computer aided design/computer aided manufacturing resource module). The CAD/CAM resource module **324** may be a dental software component that retrieves data from individual phases of a dental software workflow and/or prepares said data for use as CAD/CAM input data **320.** For example, through access to a 3D model of a patient's jaw obtained through a 3D scan with dental scanner **122** in a scanning phase, CAD/CAM resource module **324** may automatically derive, through image analysis identifying a scan body, a "tooth number" representative of a tooth to be restored, thus preventing the practitioner from needing to manually enter said tooth number. Said "tooth number" may thus become part of input data **302.** In another example, the CAD/CAM resource module **324** automatically detects a drilled cavity in a scanned tooth, by for example, image analysis and derives an "inlay" as a type of restoration to be designed. Thus, an "inlay" becomes a part of input data **302.** Further, the CAD/CAM resource module **324** detects from preferences **322** obtained from, for example, patient profile, practitioner profile, group profile, connected devices etc. From a patient profile, an esthetic material may be preferred. Normally, **1-3** mm of maxillary incisal tooth structure shows at rest in a youthful smile. From this position, if the patient has a high esthetic demand and shows a great deal of tooth structure (more than **7** mm of lip hypermobility when smiling, according to the patient's profile), a material that is as cosmetic as possible may usually be chosen as input to a machine learning engine in order to obtain dental parameter output proposals. However, the CAD/CAM resource module **324,** having access to the patient's profile or scan data detects evidence of occlusal-muscle disorders, masticatory muscle soreness or fatigue (tension headaches), TMJ (temporomandibular joint) issues, tooth wear, etc. Since these issues may affect the appropriateness of output proposals, they may form part of a set of dependencies **326** to be used as input data for proposals generation. Other dependencies include, for example, whether an "extra fine" machining mode is compatible with sinterable materials or whether a late change to a restoration design in a dental software maintains restoration integrity with or without a corresponding change in a predefined property (e.g. whether a late change can taking into consideration margin enforcement). Further, individual portions of the input data **302** may be weighted or prioritized to drive corresponding changes in proposals. These dependencies may be complex, need significant education and may not always be accessible to every user. Thus, not taking said dependencies into account may result in sub-optimal machining results, processes and cost.

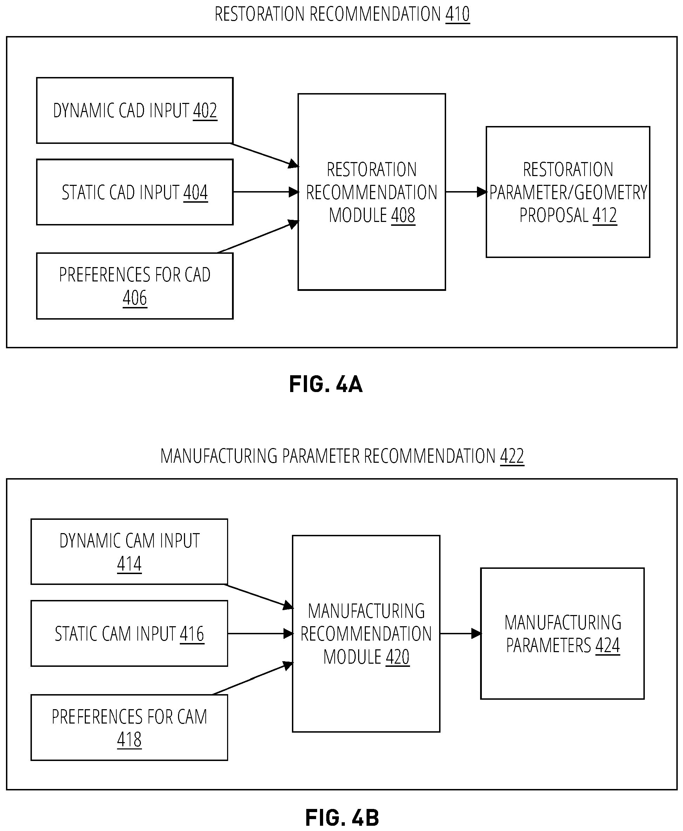

In an embodiment, feature extraction component **314** is configured to generate relevant features for a proposal based on data from all the different available features (e.g., CAD/CAM input data **320,** preferences **322**). In the embodiment, feature extraction component **314** receives a request from application **304** which includes at least an identification of a recommendation/proposal type needed (e.g. Restoration recommendation **410** or manufacturing parameter recommendation **422** as shown in FIG. 4A and FIG. 4B). Based on the recommendation type, feature extraction component **314** obtains any combination of specific CAD/CAM input data **320,** patient, practitioner or group profile information from preferences **322** and/or any dependencies **326** that are relevant to the request or proposal needed. In the embodiment, feature extraction component **314** uses a defined algorithm of prioritization or dependencies to generate the features as input for the configuration recommendation module **316.** In a particular embodiment, as shown in FIG. 4A, the recommendation type is a restoration recommendation **410** and the extracted features include dynamic CAD input **402,** static CAD input **404** and preferences for CAD **406.** The dynamic CAD input **402** or patient-specific CAD input for restoration recommendation **410** includes, for example, a 3D geometry of the patient's jaw, a tooth number for a tooth to be restored, and a restoration type to be produced. The static CAD input **404** for restoration recommendation **410** include, for example, milling unit/3D printer, material type, and instruments/tools connected to the dental system, whereas the preferences for CAD **406** include, for example, esthetic quality. The proposals for restoration recommendations **410** may include a CAD restoration geometry and restoration parameters such as a minimum wall thickness value, an edge thickness value, margin line proposal, an edge design (chamfer or rounded, chamfer length, edge radius), a spacer design etc., referred to herein as restoration parameter/geometry proposals **412.** In another particular embodiment, as shown in FIG. 4B, the recommendation type is a manufacturing parameter recommendation **422** and the extracted features include dynamic CAM input **414,** static CAM input **416** and preferences for CAM **418.** The dynamic CAM input **414** or patient-specific CAM input for manufacturing parameter recommendations **422** includes, for example, a restoration geometry such as a proposed restoration geometry from the restoration recommendation **410** , a tooth number for a tooth to be restored, and a type of the proposed restoration (e.g. crown, inlay, onlay, veneer, etc.). The static CAM input **416** for manufacturing parameter recommendation **422** includes, for example, milling unit/3D printer, material type, and instruments/tools connected to the dental system, whereas the preferences for CAM **418** for manufacturing parameter recommendation **422** include, for example, esthetic quality and milling time. The proposals for manufacturing parameter recommendation **422** may include manufacturing parameters **424** such as parameters to be used during machining e.g. choices for process definitions (including preforms, roughing, finishing, fine finishing, post-processing of fissure, post-processing of preparation line, pin reduction, variation of insertion axis, decision about **3** axes or **5** axes, simultaneous machining), choices for path strategies (including constant-z, constant-x/y, constant cusp, slicing) choices for path distances, feed sizes, form feeds, acceleration limitation, back limitation, revolution speed, revolution direction etc. The manufacturing parameters **424** may also include parameters to be used during 3D printing such as positioning and orientation in a building space, fused deposition modeling (FDM), thread width, printing slice size, image resolution, exposure intensity, time etc.

In one of more illustrative embodiments, the feature extraction component **314** is incorporated in a deep neural network. The configuration recommendation module **316** uses the obtained features to generate proposals **312.** The configuration recommendation module **316** can be based, for example, on an artificial machine learning neural network such as a convolutional neural network (CNN), though it is not meant to be limiting. It is a feed-forward artificial neural network which in a classic form consists of a convolutional layer, followed by a pooling layer. The CNN learns by learning free parameters or classifiers of the convolution kernel per layer and their weighting when calculating the next layer. A training of the m/l model **306** or configuration recommendation module **316** according to an illustrative embodiment is discussed hereinafter.

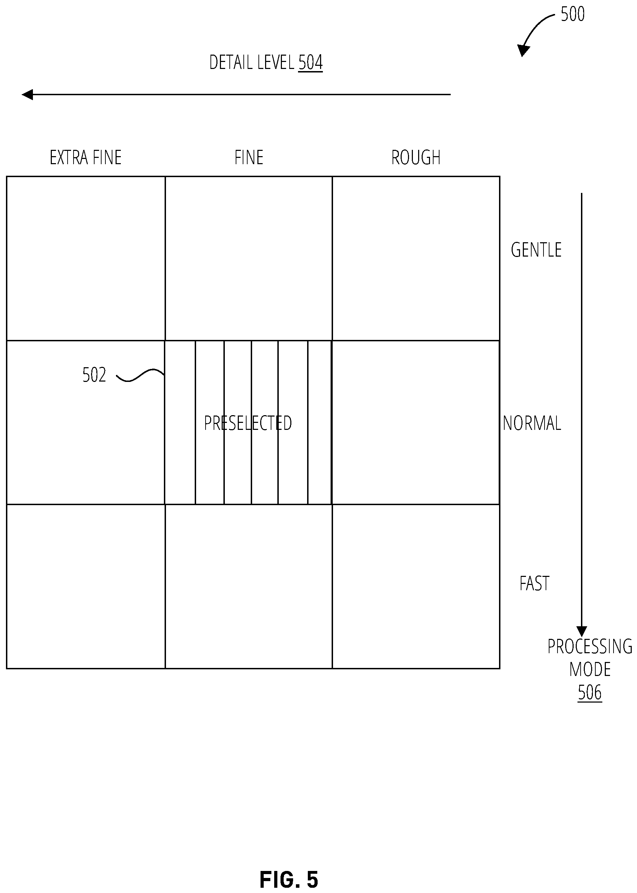

In an illustrative embodiment, presentation module **308** of application **304** displays proposals obtained from the configuration recommendation module **316.** The presentation module **308** may display, for example, a slider showing a range of machining speeds with a proposed machining speed being pre-selected for the practitioner. In another example, a proposed minimum wall thickness is displayed or drawn on a restoration design. An adaptation module **310** is configured to receive input from the practitioner to adapt the proposals **312** if necessary. For example, changing a proposed margin line may cause recalculation of a proposed restoration geometry for presentation by the presentation module **308.** In an illustrative embodiment, the presentation module **308** presents a machining interface **500,** as shown in FIG. 5, illustrating a set of a detail level **504** and processing mode **506** options. A proposed option may be preselected **502** based on the output of the configuration recommendation module **316.** Detail level **504,** which in an illustrative embodiment represents the level of detail of structures such as pits and fissures to be produced on a manufactured restoration depends on the type of tools used in a milling process. For example, an "Cylinder Pointed Bur 12 EF" bur may be needed to produce a "extra fine" detail level on a rightward side of a bridge restoration but said tool may not be available at a practitioner's office and thus a fine detail level may be preselected **502.** Further, a "fast" milling unit processing mode **506** may not be suitable for manufacturing a posterior bridge and thus a "normal" processing mode is preselected **502** for the patient-specific case. Further, a "fast" processing mode may not be an option for a zirconia restoration unless a corresponding margin thickness parameter is **100** microns, for example, or higher. Zirconia restorations with less than **100** microns of margin thickness are more prone to undesired chipping. This may therefore become a dependency that is used by the configuration recommendation module **316** for proposals. As new tools and materials with specific instructions and requirements are added to a dental workflow, a need for the practitioner to be intimately aware of these instructions and requirements is significantly reduced by the use of the configuration recommendation module **316** which is configured to locally or remotely take the new instructions and requirements in one or more forms, generally referred to herein as dependencies **326,** into consideration to produce optimized proposals.

Feedback module **318** optionally collects user feedback **322** relative to the proposals **312.** In one embodiment, application **304** is configured not only to present recommendations **410** and **412** but also to provide a method for a dental practitioner to input a feedback, where the feedback is indicative of an accuracy of the recommendation. Feedback module **318** applies the feedback in a machine learning technique such as to dependencies **326** or user profiles to modify the m/l model **306** used in the configuration recommendation module **316.**

In an illustrative embodiment, after proposing a recommendation, the application **304** may perform additional operations automatically or in response to a request. Based on the available manufacturing devices and preferences of the user automatic steps may be defined including a) an automatic generation of the ideal restoration based on material, hardware, instruments/tools and user preferences (level of detail of fissures etc.). b) automatic optimization of the restoration parameters (minimum wall thickness, level of detail, edge enhancement etc.) for the a preferred machining method (grinding, milling, printing, tools, machining detail level (fine, fast, extra fine, extra fast etc.) and a given restoration geometry, c) automatic positioning inside of a machining medium (e.g. a block) of a dental manufacturing system for optimal use of the material (minimal usage of material / tool, correct color at block with color gradient) with best possible quality / performance, optimal positioning and size of tapping place, i.e. position of the pin, where the restoration is mounted to the block holder before cutting off, (the size depends on machining type, device and material), d) wear status of the tools taken into account for machining job, and e) consideration of user preferences for setting of properties. Of course, these are merely non-limiting examples and other examples are possible based on descriptions herein.

The input layer of the neural network model can be, for example, a vector representative of a restoration type, pixels of a **2**D image, **2.5**D images having depth information and gray level information, 3D points of 3D images or 3D scans in a gird-like form etc. In an example, a first CNN uses convolution to extract features from an input image. A second CNN uses up-sampling or transpose convolution to create a prediction of how the object would look in three-dimensions, from the features extracted by the first neural network.

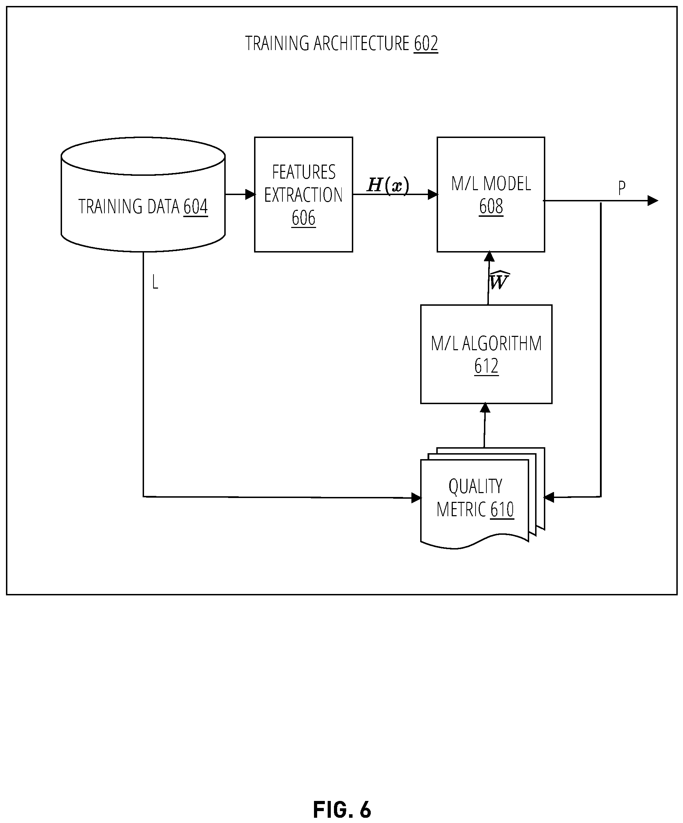

The neural network m/l model **306** is trained using various types of training data sets including static and dynamic CAD and CAM inputs as well as preferences. In an embodiment, upon receiving a request to provide a recommendation, application **304** creates an array of values that are input to the input neurons of the m/l model **306** to produce an array that contains the recommendation/proposal **312.** As shown in FIG. 6, which depicts a block diagram of an example training architecture **602** for machine-learning based recommendation generation in accordance with an illustrative embodiment, program code extracts various features/attributes **606** from training data **604** with the training data entries having labels L. The features are utilized to develop a predictor function, H(x) or a hypothesis, which the program code utilizes as a machine learning model **608.** In identifying various features/attributes in the training data **604,** the program code may utilize various techniques including, but not limited to, mutual information, which is an example of a method that can be utilized to identify features in an embodiment. Other embodiments may utilize varying techniques to select features, including but not limited to, principal component analysis, diffusion mapping, a Random Forest, and/or recursive feature elimination (a brute force approach to selecting features), to select the features. "P" is the output (e.g., restoration or dental parameters) that can be obtained, which when received, could further trigger the dental system to perform other steps such as display an element of start a machining process. The program code may utilize a machine learning algorithm **612** to train machine learning model **608,** including providing weights for the outputs, so that the program code can prioritize various changes based on the predictor functions that comprise the machine learning model **608.** The output can be evaluated by a quality metric **610.**

By selecting a diverse set of training data **604,** the program code trains machine learning model **608** to identify and weight various attributes of patients, practitioners, devices connected to the dental system, etc. To utilize the machine learning model **608,** the program code obtains (or derives) input data or features to generate an array of values to input into input neurons of a neural network. Responsive to these inputs, the output neurons of the neural network produce an array that includes the proposals **312** to be presented contemporaneously on a display.

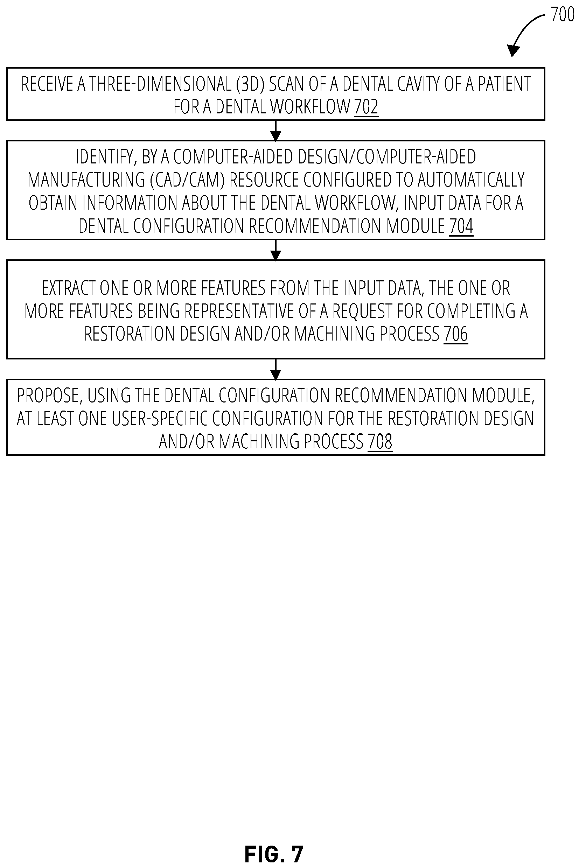

With reference to FIG. 7, this figure depicts a flowchart of an example process **700** for configuring dental workflows. Process **700** can be implemented using application **304** of FIG. 3. The application **304** receives a three-dimensional (3D) scan of a dental cavity of a patient for a dental workflow, step **702.** In step **704,** the application **304** identifies, by a CAD/CAM resource module **324** configured to automatically obtain information about the dental workflow, input data for the dental configuration recommendation module **316.** In step **706,** the application **304** extracts, responsive to the identifying step, one or more features from the input data. The one or more features are representative of a request for completing a restoration design and/or a machining process. In step **708,** the application **304** proposes, using the dental configuration recommendation module **316,** at least one user-specific configuration for the restoration design and/or machining process. In step **708,** the proposing can include at least one of: the proposal of a user-specific restoration design, at least one restoration parameter and/or at least one user-specific manufacturing parameter. After presenting the proposal, a user such as a practitioner may use an adaptation module to modify one or more proposals or one or more defined settings in the dental workflow process and the modification may be accepted or denied upon checking that the modification does not cause unintended consequences such as a reduction in the integrity of any restoration to be manufactured or a non-adherence to margin line restrictions. The acceptance may be determined based on a defined set of integrity rules or dependencies between parameters. The modification may also cause a need to re-compute proposals or designs, for example contemporaneously in order to take the modifications into consideration.

Further, the application **304** may receive a user feedback input relative. The application analyzes the said feedback input and the application reinforces the m/l model **306** of the configuration recommendation module **316.** If the feedback is satisfactory or unsatisfactory as to the accuracy of the proposal, the application strengthens or weakens parameters of the m/l model **306** respectively. The application **304** ends the process **700** thereafter.

Thus, a computer implemented method, system or apparatus, and computer program product are provided in the illustrative embodiments for intelligent dental workflow configurations and other related features, functions, or operations. Where an embodiment or a portion thereof is described with respect to a type of device, the computer implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser (e.g., web-based e-mail), or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

Disclosed is also a computer
program product at any possible technical detail level of integration. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory **(RAM),** a read-only memory **(ROM),** an erasable programmable read-only memory **(EPROM** or Flash memory), a static random access memory **(SRAM),** a portable compact disc read-only memory **(CD-ROM),** a digital versatile disk **(DVD),** a memory stick, a floppy disk, a mechanically encoded device such as punch-cards or raised structures in a groove having instructions recorded thereon, and any suitable combination of the foregoing. A computer readable storage medium, including but not limited to computer-readable storage devices as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, Python, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A computer-implemented method comprising the steps of:
receiving a three-dimensional (3D) scan of a dental cavity of a patient for a dental workflow;
identifying, by a computer-aided design/computer-aided manufacturing (CAD/CAM) resource configured to automatically obtain information about the dental workflow (702), input data for a dental configuration recommendation module (704);
extracting, responsive to the identifying step, one or more features from the input data, the one or more features representative of a request for completing a restoration design and/or machining process (706);
proposing, using the dental configuration recommendation module (704), at least one configuration for the restoration design and/or machining process (706); and
providing feedback for the dental configuration recommendation module (704) indicative of an accuracy of proposals in order to reinforce the dental configuration recommendation module (704).

2. The method of claim 1, wherein said proposing step includes at least one of (a) proposing a user-specific 3D restoration design as a first configuration, (b) proposing at least one user-specific restoration parameter as a second configuration, and (c) proposing at least one user-specific manufacturing parameter as a third configuration.

3. The method of claim 2, further comprising the steps of:
providing, using an adaptation module, a modification of a dental workflow setting or of a proposed user-specific configuration from a plurality of proposed user-specific configurations and computing an acceptance decision of the modification based on a set of dependencies.

4. The method of claim 3, further comprising the step of:
recomputing, the at least one user-specific configuration upon determining that the modification is acceptable.

5. The method of claim 1, wherein the input data is automatically obtained from devices contained in or connected to the dental manufacturing system.

6. The method of claim 1, wherein at least a portion of the input data is automatically retrieved from the 3D scan of the dental cavity.

7. The method of claim 1, wherein the dental configuration recommendation module operates as a machine learning engine.

8. The method of claim 7, further comprising the step of:
training the dental configuration recommendation module based on information comprising static CAD/CAM input data, dynamic CAD/CAM input data and user preferences.

9. The method of claim 8, wherein the information includes historical data selected from a database of a dental manufacturing system.

10. The method of claim 1, wherein the input data is data being selected from the group consisting of CAD/CAM input data, preferences and dependencies.

11. The method of claim 10, further comprising the step of locally or remotely providing the dental configuration recommendation module with new instructions and requirements as the dependencies.

12. A computer system comprising a processor configured to perform the steps including:
receiving a three-dimensional (3D) scan of a dental cavity of a patient for a dental workflow;
identifying, by a computer-aided design/computer-aided manufacturing (CAD/CAM) resource configured to automatically obtain information about the dental workflow (702), input data for a dental configuration recommendation module (704);
extracting, responsive to the identifying step, one or more features from the input data, the one or more features representative of a request for completing a restoration design and/or machining process (706);
proposing, using the dental configuration recommendation module (704), at least one configuration for the restoration design and/or machining process (706); and
providing feedback for the dental configuration recommendation module (704) indicative of an accuracy of proposals in order to reinforce the dental configuration recommendation module (704).

13. The computer system of claim 12, wherein the dental configuration recommendation module operates as a machine learning engine.

14. A non-transitory computer-readable storage medium storing a program which, when executed by a computer system, causes the computer system to perform a procedure comprising the steps of:
receiving a three-dimensional (3D) scan of a dental cavity of a patient for a dental workflow;
identifying, by a computer-aided design/computer-aided manufacturing (CAD/CAM) resource configured to automatically obtain information about the dental workflow, (702) input data for a dental configuration recommendation module (704);
extracting, responsive to the identifying step, one or more features from the input data, the one or more features representative of a request for completing a restoration design and/or machining process (706);
proposing, using the dental configuration recommendation module (704), at least one configuration for the restoration design and/or machining process (706); and
providing feedback for the dental configuration recommendation module (704) indicative of an accuracy of proposals in order to reinforce the dental configuration recommendation module (704).

## Patentansprüche

1. Computerimplementiertes Verfahren, umfassend die folgenden Schritte:
Empfangen eines dreidimensionalen (3D) Scans einer Zahnkavität eines Patienten für einen Zahnarbeitsablauf;
Identifizieren, durch eine computergestützte Entwurfs-/computergestützte Fertigungs-(CAD/CAM) Ressource, die dazu konfiguriert ist, automatisch Informationen über den Zahnarbeitsablauf (702) zu erhalten, von Eingabedaten für ein Zahnkonfigurationsempfehlungsmodul (704);
Extrahieren, als Reaktion auf den Identifizierungsschritt, eines oder mehrerer Merkmale aus den Eingabedaten, wobei das eine oder die mehreren Merkmale für eine Anforderung zum Abschließen eines Wiederherstellungsentwurfs und/oder eines Bearbeitungsprozesses (706) repräsentativ sind;
Vorschlagen, unter Verwendung des Zahnkonfigurationsempfehlungsmoduls (704), mindestens einer Konfiguration für den Wiederherstellungsentwurf und/oder den Bearbeitungsprozess (706); und
Bereitstellen von Feedback für das Zahnkonfigurationsempfehlungsmodul (704), das auf eine Genauigkeit von Vorschlägen hinweist, um das Zahnkonfigurationsempfehlungsmodul (704) zu verstärken.

2. Verfahren nach Anspruch 1, wobei der Vorschlagsschritt mindestens eines von (a) Vorschlagen eines benutzerspezifischen 3D-Wiederherstellungsentwurfs als eine erste Konfiguration, (b) Vorschlagen mindestens eines benutzerspezifischen Wiederherstellungsparameters als eine zweite Konfiguration und (c) Vorschlagen mindestens eines benutzerspezifischen Fertigungsparameters als eine dritte Konfiguration beinhaltet.

3. Verfahren nach Anspruch 2, ferner umfassend die folgenden Schritte:
Bereitstellen, unter Verwendung eines Anpassungsmoduls, einer Modifikation einer Zahnarbeitsablaufeinstellung oder einer vorgeschlagenen benutzerspezifischen Konfiguration aus einer Vielzahl von vorgeschlagenen benutzerspezifischen Konfigurationen und Berechnen einer Annahmeentscheidung der Modifikation basierend auf einem Satz von Abhängigkeiten.

4. Verfahren nach Anspruch 3, ferner umfassend den folgenden Schritt:
Neuberechnen der mindestens einen benutzerspezifischen Konfiguration, wenn bestimmt wird, dass die Modifikation annehmbar ist.

5. Verfahren nach Anspruch 1, wobei die Eingabedaten automatisch von Vorrichtungen erhalten werden, die in dem Zahnfertigungssystem enthalten oder mit diesem verbunden sind.

6. Verfahren nach Anspruch 1, wobei zumindest ein Teil der Eingabedaten automatisch von dem 3D-Scan der Zahnkavität abgerufen wird.

7. Verfahren nach Anspruch 1, wobei das Zahnkonfigurationsempfehlungsmodul als eine Maschine zum maschinellen Lernen arbeitet.

8. Verfahren nach Anspruch 7, ferner umfassend den folgenden Schritt:
Trainieren des Zahnkonfigurationsempfehlungsmoduls basierend auf Informationen, die statische CAD/CAM-Eingabedaten, dynamische CAD/CAM-Eingabedaten und Benutzerpräferenzen umfassen.

9. Verfahren nach Anspruch 8, wobei die Informationen historische Daten beinhalten, die aus einer Datenbank eines Zahnfertigungssystems ausgewählt sind.

10. Verfahren nach Anspruch 1, wobei die Eingabedaten Daten sind, die aus der Gruppe ausgewählt sind, die aus CAD/CAM-Eingabedaten, Präferenzen und Abhängigkeiten besteht.

11. Verfahren nach Anspruch 10, ferner umfassend den Schritt zum lokalen oder entfernten Versehens des Zahnkonfigurationsempfehlungsmoduls mit neuen Anweisungen und Anforderungen als die Abhängigkeiten.

12. Computersystem, umfassend einen Prozessor, der dazu konfiguriert ist, die Schritte durchzuführen, die Folgendes beinhalten: Empfangen eines dreidimensionalen (3D) Scans einer Zahnkavität eines Patienten für einen Zahnarbeitsablauf;
Identifizieren, durch eine computergestützte Entwurfs-/computergestützte Fertigungs-(CAD/CAM) Ressource, die dazu konfiguriert ist, automatisch Informationen über den Zahnarbeitsablauf (702) zu erhalten, von Eingabedaten für ein Zahnkonfigurationsempfehlungsmodul (704);
Extrahieren, als Reaktion auf den Identifizierungsschritt, eines oder mehrerer Merkmale aus den Eingabedaten, wobei das eine oder die mehreren Merkmale für eine Anforderung zum Abschließen eines Wiederherstellungsentwurfs und/oder eines Bearbeitungsprozesses (706) repräsentativ sind;
Vorschlagen, unter Verwendung des Zahnkonfigurationsempfehlungsmoduls (704), mindestens einer Konfiguration für den Wiederherstellungsentwurf und/oder den Bearbeitungsprozess (706); und
Bereitstellen von Feedback für das Zahnkonfigurationsempfehlungsmodul (704), das auf eine Genauigkeit von Vorschlägen hinweist, um das Zahnkonfigurationsempfehlungsmodul (704) zu verstärken.

13. Computersystem nach Anspruch 12, wobei das Zahnkonfigurationsempfehlungsmodul als eine Maschine zum maschinellen Lernen arbeitet.

14. Nichtflüchtiges computerlesbares Speichermedium, das ein Programm speichert, das, wenn es von einem Computersystem ausgeführt wird, bewirkt, dass das Computersystem eine Prozedur durchführt, die die folgenden Schritte umfasst:
Empfangen eines dreidimensionalen (3D) Scans einer Zahnkavität eines Patienten für einen Zahnarbeitsablauf;
Identifizieren, durch eine computergestützte Entwurfs-/computergestützte Fertigungs-(CAD/CAM) Ressource, die dazu konfiguriert ist, automatisch Informationen über den Zahnarbeitsablauf (702) zu erhalten, von Eingabedaten für ein Zahnkonfigurationsempfehlungsmodul (704);
Extrahieren, als Reaktion auf den Identifizierungsschritt, eines oder mehrerer Merkmale aus den Eingabedaten, wobei das eine oder die mehreren Merkmale für eine Anforderung zum Abschließen eines Wiederherstellungsentwurfs und/oder eines Bearbeitungsprozesses (706) repräsentativ sind;
Vorschlagen, unter Verwendung des Zahnkonfigurationsempfehlungsmoduls (704), mindestens einer Konfiguration für den Wiederherstellungsentwurf und/oder den Bearbeitungsprozess (706); und
Bereitstellen von Feedback für das Zahnkonfigurationsempfehlungsmodul (704), das auf eine Genauigkeit von Vorschlägen hinweist, um das Zahnkonfigurationsempfehlungsmodul (704) zu verstärken.

## Revendications

1. Procédé mis en œuvre par ordinateur comprenant les étapes de :
réception d'un balayage tridimensionnel (3D) d'une cavité dentaire d'un patient pour un flux de travail dentaire ;
identification, par une ressource de conception assistée par ordinateur/fabrication assistée par ordinateur (CAO/FAO) configurée pour obtenir automatiquement des informations concernant le flux de travail dentaire (702), des données d'entrée pour un module de recommandation de configuration dentaire (704) ;
extraction, en réponse à l'étape d'identification, d'une ou plusieurs caractéristiques des données d'entrée, la ou les caractéristiques représentant une demande d'achèvement d'un processus de conception et/ou d'usinage de restauration (706) ;
proposition, à l'aide du module de recommandation de configuration dentaire (704), d'au moins une configuration pour le processus de conception et/ou d'usinage de restauration (706) ; et
fourniture d'une rétroaction pour le module de recommandation de configuration dentaire (704) indiquant une précision des propositions dans le but de renforcer le module de recommandation de configuration dentaire (704).

2. Procédé de la revendication 1, ladite étape de proposition comprenant au moins un parmi (a) la proposition d'une conception de restauration 3D spécifique à l'utilisateur en tant que première configuration, (b) la proposition d'au moins un paramètre de restauration spécifique à l'utilisateur en tant que deuxième configuration, et (c) la proposition d'au moins un paramètre de fabrication spécifique à l'utilisateur en tant que troisième configuration.

3. Procédé de la revendication 2, comprenant en outre les étapes de :
fourniture, à l'aide d'un module d'adaptation, d'une modification d'un paramètre de flux de travail dentaire ou d'une configuration spécifique à l'utilisateur proposée à partir d'une pluralité de configurations spécifiques à l'utilisateur proposées et le calcul d'une décision d'acceptation de la modification sur la base d'un ensemble de dépendances.

4. Procédé de la revendication 3, comprenant en outre l'étape de :
calcul à nouveau de l'au moins une configuration spécifique à l'utilisateur lors de la détermination que la modification est acceptable.

5. Procédé de la revendication 1, lesdites données d'entrée étant automatiquement obtenues à partir de dispositifs contenus dans le système de fabrication dentaire ou connectés à celui-ci.

6. Procédé de la revendication 1, au moins une partie des données d'entrée étant automatiquement récupérée à partir du balayage 3D de la cavité dentaire.

7. Procédé de la revendication 1, ledit module de recommandation de configuration dentaire fonctionnant en tant que moteur d'apprentissage automatique.

8. Procédé de la revendication 7, comprenant en outre l'étape de :
formation du module de recommandation de configuration dentaire sur la base d'informations comprenant des données d'entrée CAO/FAO statiques, des données d'entrée CAO/FAO dynamiques et des préférences de l'utilisateur.

9. Procédé de la revendication 8, lesdites informations comprenant des données historiques sélectionnées à partir d'une base de données d'un système de fabrication dentaire.

10. Procédé de la revendication 1, lesdites données d'entrée étant des données sélectionnées dans le groupe constitué de données d'entrée CAO/FAO, de préférences et de dépendances.

11. Procédé de la revendication 10, comprenant en outre l'étape de fourniture locale ou à distance au module de recommandation de configuration dentaire de nouvelles instructions et exigences en tant que dépendances.

12. Système informatique comprenant un processeur configuré pour réaliser les étapes comprenant :
la réception d'un balayage tridimensionnel (3D) d'une cavité dentaire d'un patient pour un flux de travail dentaire ;
l'identification, par une ressource de conception assistée par ordinateur/fabrication assistée par ordinateur (CAO/FAO) configurée pour obtenir automatiquement des informations concernant le flux de travail dentaire (702), des données d'entrée pour un module de recommandation de configuration dentaire (704) ;
extraction, en réponse à l'étape d'identification, d'une ou plusieurs caractéristiques des données d'entrée, la ou les caractéristiques représentant une demande d'achèvement d'un processus de conception et/ou d'usinage de restauration (706) ;
la proposition, à l'aide du module de recommandation de configuration dentaire (704), d'au moins une configuration pour le processus de conception et/ou d'usinage de restauration (706) ; et
la fourniture d'une rétroaction pour le module de recommandation de configuration dentaire (704) indiquant une précision de propositions dans le but de renforcer le module de recommandation de configuration dentaire (704).

13. Système informatique de la revendication 12, ledit module de recommandation de configuration dentaire fonctionnant en tant que moteur d'apprentissage automatique.

14. Support de stockage lisible par ordinateur non transitoire stockant un programme qui, lorsqu'il est exécuté par un système informatique, amène le système informatique à réaliser une procédure comprenant les étapes de :
réception d'un balayage tridimensionnel (3D) d'une cavité dentaire d'un patient pour un flux de travail dentaire ;
l'identification, par une ressource de conception assistée par ordinateur/fabrication assistée par ordinateur (CAO/FAO) configurée pour obtenir automatiquement des informations concernant le flux de travail dentaire (702), des données d'entrée pour un module de recommandation de configuration dentaire (704) ;
extraction, en réponse à l'étape d'identification, d'une ou plusieurs caractéristiques des données d'entrée, la ou les caractéristiques représentant une demande d'achèvement d'un processus de conception et/ou d'usinage de restauration (706) ;
la proposition, à l'aide du module de recommandation de configuration dentaire (704), d'au moins une configuration pour le processus de conception et/ou d'usinage de restauration (706) ; et
la fourniture d'une rétroaction pour le module de recommandation de configuration dentaire (704) indiquant une précision de propositions dans le but de renforcer le module de recommandation de configuration dentaire (704).
